# EUROPEAN PATENT APPLICATION

(11) **EP 4 570 282 A1**
(43) Date of publication of application: **18.06.2025**
(21) Application number: 24219403.3
(22) Date of filing: 12.12.2024
(51) Int. Cl.: A61M 5/142, H04R 1/02, H04R 1/30, H04R 17/00

(54) **SOUND PROPAGATION IN AN ENCLOSED DEVICE**

(30) Priority: 14.12.2023 US 202363610362 P; 10.12.2024 US 202418975268
(71) Applicant: Medtronic MiniMed, Inc., Northridge, CA 91325 (US)
(72) Inventor: Thompson, Chase A., Northridge, 91325 (US); Kang, Min Seung, Northridge, 91325 (US); Cheney II, Paul S., Northridge, 91325 (US); Miya, Isabella, Northridge, 91325 (US); Tucker, Derek E., Austin, 78752 (US); Brown, Lorne, 9777407 Jerusalem (IL); Frie, Euphemia, Northridge, 91325 (US)
(74) Representative: Zimmermann & Partner Patentanwälte mbB

(57) **Abstract**

In general, the disclosure describes techniques for generating sounds in fully sealed medical devices. The example techniques for generating sounds in medical devices include the use of a plurality of sound chambers arranged in a stacked configuration that propagate the sound, along with one or more diaphragms configured to vibrate in response to the sound propagating through the medical device. In one or more examples, an external housing that includes an overlay cover may fully enclose the medical device, where the overlay cover (e.g., the entire overlay cover or a part of the overlay cover) also vibrates to propagate the sound out of the medical device.

## Description

### FIELD

This disclosure generally relates to systems including medical devices and, more particularly, to generating audible alerts using such systems.

### BACKGROUND

Certain diseases or conditions may be treated, according to modern medical techniques, by delivering a medication or other substance to the body of a user, either in a continuous manner or at particular times or time intervals within an overall time period. For example, diabetes is commonly treated by delivering defined amounts of insulin to the user at appropriate times. Some modes of providing insulin therapy to a user include delivery of insulin through manually operated syringes and insulin pens. Some other modes employ programmable fluid infusion devices (e.g., insulin pumps) to deliver controlled amounts of insulin to a user.

Fluid infusion devices include devices designed for use in a generally stationary location (for example, in a hospital or clinic), and devices configured for ambulatory or portable use (to be carried by a user). A fluid flow path may be established from a fluid reservoir of a fluid infusion device to the user via, for example, a set connector of an infusion set, which is coupled to the fluid reservoir. Insulin pumps are generally designed to be waterproof, such that a user may wear the insulin pump while maintaining a normal daily routine, which may include contact with water (e.g., rain, shower, etc.). Insulin pumps also may require an audible alarm to notify a user of various conditions, such as fault conditions (e.g., low battery, low insulin levels, communication errors, etc.). However, a waterproof device is often not conducive to enabling sound to efficiently propagate from a fully sealed device.

### SUMMARY

In general, the disclosure describes techniques for generating sounds in fully sealed medical devices. The example techniques for generating sounds in medical devices include the use of a plurality of sound chambers arranged in a stacked configuration that propagate the sound, along with one or more diaphragms configured to vibrate in response to the sound propagating through the medical device. In one or more examples, an external housing that includes an overlay cover may fully enclose the medical device, where the overlay cover (e.g., the entire overlay cover or a part of the overlay cover) also vibrates to propagate the sound out of the medical device.

With the plurality of sound chambers arranged in the stacked configuration, the one or more diaphragms, and the overlay cover, the example medical devices may be configured to generate sound having sufficiently high decibels (dBs) that propagates out of the medical device even when the medical device is fully enclosed. For example, in the stacked configuration, the sound may propagate serially through the sound chambers, and therefore limit loss as compared to non-stacked configurations. Furthermore, it may be advantageous for a medical device to have ingress protection, making it possible to use the device in varying conditions, e.g., outdoors. As an example, wearable fluid infusion devices are carried by users constantly and may require frequent use, and diabetic users may be able to perform some activities of daily living more easily with a fully sealed fluid infusion device.

The example techniques are described with respect to a medical device, such as a fluid infusion device. However, the example techniques are applicable to other types of medical devices as well. Also, the example techniques should not be considered limited to medical devices and may be applicable to non-medical devices as well.

In one example, a medical device comprising: a speaker comprising a vibrating membrane and configured to generate a sound that propagates through the medical device; a plurality of sound chambers in a stacked configuration and configured to propagate the sound generated by the speaker through one or more sound tuning holes of the sound chambers, wherein in the stacked configuration the sound propagates serially through the plurality of sound chambers; a one or more diaphragms, within the medical device, configured to vibrate in response to the sound propagating though the medical device, wherein each of the one or more diaphragms separates respective sound chambers in the stacked configuration and vibrates in response to the sound propagating from a first sound chamber of the respective sound chambers to a second sound chamber of the respective sound chambers; an external housing fully enclosing the medical device and comprising an overlay cover, wherein the overlay cover is configured to vibrate in response to the sound propagating through the plurality of sound chambers in the stacked configuration.

In another example, a method of generating an audible alert comprising: determining that an audible alert is to be output; and upon determining that the audible alert is to be output, causing a speaker comprising a vibrating membrane to generate a sound that propagates through the medical device through a plurality of sound chambers in a stacked configuration and configured to propagate the sound generated by the speaker through one or more sound tuning holes of the sound chambers, wherein in the stacked configuration the sound propagates serially through the plurality of sound chambers, wherein the sound chambers in the stacked configuration are separated by one of one or more diaphragms and each of the one or more diaphragms vibrates in response to the sound propagating from a first sound chamber of the respective sound chambers to a second sound chamber of the respective sound chambers, wherein an external housing fully encloses the medical device and comprises an overlay cover, wherein the overlay cover is configured to vibrate in response to the sound propagating through the plurality of sound chambers in the stacked configuration.

In another example, a method of manufacturing a medical device, the method comprising: forming a first sound chamber of a plurality of sound chambers in a stacked configuration, wherein the plurality of sound chambers is configured to propagate a sound generated by a speaker through one or more sound tuning holes of sound chambers, wherein in the stacked configuration the sound propagates serially through the plurality of sound chambers; forming a first of plurality of diaphragms within the medical device; forming a second sound chamber of the plurality of sound chambers; wherein each of the one or more diaphragms is configured to vibrate in response to the sound propagating though the respective sound chambers and one of the one or more diaphragms separates respective sound chambers in the stacked configuration and vibrates in response to the sound propagating from the first sound chamber of the respective sound chambers to the second sound chamber of the respective sound chambers; and forming an external housing fully enclosing the medical device, wherein the external housing comprises an overlay cover, wherein the overlay cover is configured to vibrate in response to the sound propagating through the plurality of sound chambers in the stacked configuration.

Further disclosed herein, in general, are techniques for generating sounds in fully sealed medical devices. The example techniques for generating sounds in medical devices include the use of a plurality of sound chambers arranged in a stacked configuration that propagate the sound, along with one or more diaphragms configured to vibrate in response to the sound propagating through the medical device. In one or more examples, an external housing that includes an overlay cover may fully enclose the medical device, where the overlay cover (e.g., the entire overlay cover or a part of the overlay cover) also vibrates to propagate the sound out of the medical device.

This summary is intended to provide an overview of the subject matter described in this disclosure. It is not intended to provide an exclusive or exhaustive explanation of the apparatus and methods described in detail within the accompanying drawings and description below. Further details of one or more examples are set forth in the accompanying drawings and the description below.

### BRIEF DESCRIPTION OF DRAWINGS

FIG. 1 is a perspective view of an exemplary external housing of a medical device, in accordance with one or more techniques of this disclosure.
FIGS. 2A-2C are diagrams illustrating cross-sectional views of exemplary configurations of stacked sound chambers in accordance with one or more techniques of this disclosure.
FIG. 3 is a bottom view of an exemplary external housing portion configured for user interaction, in accordance with one or more techniques of this disclosure.
FIG. 4 is a graphical illustration of a relationship between frequency and sound pressure level, in accordance with one or more techniques of this disclosure.
FIG. 5 is a flow diagram illustrating an exemplary operation for initiating sound propagation, in accordance with one or more techniques of this disclosure.
FIGS. 6A and 6B are diagrams depicting exemplary geometries of a thermal bonding adhesive, in accordance with one or more techniques of this disclosure.
FIG. 7 is a conceptual diagram illustrating an example sound tuning hole configuration, in accordance with one or more techniques of this disclosure.

Like reference characters refer to like elements throughout the figures and description.

### DETAILED DESCRIPTION

Many medical device systems include alert-generating components. Alerts may be generated to indicate a user state or a device state that requires user, caregiver, or clinician attention. Some medical device systems comprise a single device that generates alerts based on sensed data. Other medical device systems comprise two or more devices in communication with one another to sense data and generate alerts based on the sensed data. Sensed data may comprise one or more of physiological data or device data. Additionally, some medical device systems include a medical device configured to communicate, e.g., via Bluetooth, with a smart device, e.g., a smartphone or a smart watch.

Certain terminology may be used in the following description for the purpose of reference only, and thus are not intended to be limiting. Similarly, the terms "first", "second", and other such numerical terms referring to structures do not imply a sequence or order unless clearly indicated by the context. Such terminology may include the words specifically mentioned above, derivatives thereof, and words of similar import.

This disclosure relates to various examples of sound propagation techniques for a fluid infusion device, such as for the treatment of diabetes. The fluid infusion devices described herein provide a reduced form factor and/or a simplified user interface, which may reduce complexity and cost while making it easier for the user to carry the fluid infusion device. The non-limiting examples described below relate to medical devices used to treat diabetes (such as an insulin pump and/or an infusion set), although examples of the disclosed subject matter are not so limited. The examples of this disclosure may be used with any device requiring or benefitting from sound propagation, e.g., a smartphone. The fluid infusion device serves merely as a non-limiting example. Accordingly, the infused fluid is insulin in certain examples.

In one or more examples, the disclosure describes techniques for stacking sound chambers to propagate sound out of a fully sealed device. For example, a medical device may include a speaker with a vibrating membrane (e.g., piezoelectric speaker, electromechanical buzzer, piezoelectric buzzer). The speaker is configured to generate a sound that propagates through the medical device (e.g., such as due to an alert). The medical device also includes a plurality of sound chambers in a stacked configuration and configured to propagate the sound generated by the speaker through one or more sound tuning holes of the sound chambers. For example, in the stacked configuration the sound propagates serially through the plurality of sound chambers.

The medical device also includes one or more diaphragms which vibrate in response to the sound propagating though the medical device. Each of the one or more diaphragms may separate respective sound chambers in the stacked configuration and vibrate in response to the sound propagating from a first sound chamber of the respective sound chambers to a second sound chamber of the respective sound chambers. In one or more examples, there may be constrained volumes of air between the sounds chambers that enable respective diaphragms to vibrate in place.

With the plurality of sound chambers in the stacked configuration with respective diaphragms separating the sound chambers, as the sound propagates through the medical device, each diaphragm vibrates causing air to push to the next diaphragm, thereby enabling the sound to propagate through the medical device in a way that minimizes sound loss. Moreover, the medical device may include an external housing that fully encloses the medical device. To ensure that the sound level is sufficiently high even where the medical device is enclosed, the external housing includes an overlay cover, where the overlay cover is configured to vibrate in response to the sound propagating through the plurality of sound chambers in the stacked configuration. In this way, the medical device may be fully enclosed, but sound (e.g., from an alert) can propagate through and be audible to a user.

Various parameters may impact the ability of sound to propagate such as chamber and diaphragm shape and size, as well as material. This disclosure describes examples of shapes, sizes, and materials that facilitate the propagation of sound. In addition to achieving chamber and diaphragm shapes and sizes that optimize sound output, the techniques of this disclosure describe examples for minimizing the size of the chambers and diaphragms to promote compact device design. This minimization of chamber and diaphragm size while still propagating a sufficient sound, e.g., more than 45 dB, may be particularly advantageous for portable devices.

Current methods to produce sound from water-resistant medical devices often include a hydrophobic membrane which covers any electrical portions of the device with non-stacked sound chambers that are exposed to external air to enable sound propagation. Such current methods may result in a less rigorous water seal than may be desirable in some applications. The techniques disclosed here result in a more rigorous water seal, e.g., satisfying International Electrotechnical Commission (IEC) IP28, than may be possible in devices using the current methods for sound propagation.

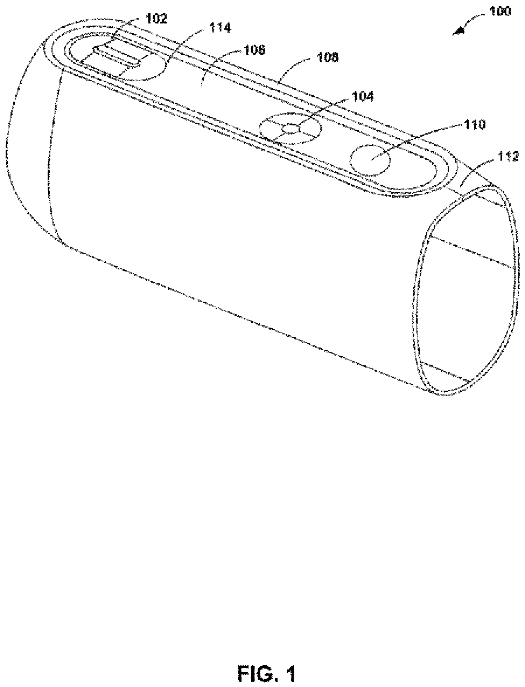

FIG. 1 is a perspective view of an exemplary external housing, in accordance with one or more techniques of this disclosure. Specifically, FIG. 1 is a perspective view of a fluid infusion device 100. In this example, the fluid infusion device 100 includes a housing 108. Generally, the housing 108 has a small form factor for portability and is about 3 inches (in.) to about 4 inches (in.) long, about 1 (in.) to about 2 inches (in.) wide and is about 0.5 inches (in.) to about 1.5 inches (in.) thick. The fluid infusion device 100 also generally weighs less than about 80 grams (g). In some examples, the housing 108 includes a first housing portion 112 and a second housing portion 106, which are coupled together to form the housing 108. In some examples, the first housing portion 112 of the housing 108 is composed of a plastic with properties including chemical resistance, durability, and impact resistance. In other examples, the first housing portion 112 of the housing 108 is composed of a metal or metal alloy, such as aluminum, titanium, stainless steel, etc., and is formed via casting, stamping, additive manufacturing, etc. By forming the first housing portion 112 of the housing 108 using a plastic, a metal, or a metal alloy, the first housing portion 112 of the housing 108, which is larger than the second housing portion 106, is resistant to environmental factors and chemical exposure, such as water, sunscreen, etc. The use of a plastic, metal, or metal alloy also protects the fluid infusion device 100 from accidental drops, vibrations, and static loads during use, which improves reliability. Moreover, the size and configuration of the housing 108 enables the fluid infusion device 100 to be carried more easily, and to be attached in different orientations, such as lengthwise, via a clip, for example. Thus, the fluid infusion device 100 is sized and shaped to enable ease of use, which increases user satisfaction and convenience.

The second housing portion 106 of the housing 108 is received within a channel of the first housing portion 112 such that the first housing portion 112 surrounds a majority of the second housing portion 106. The first housing portion 112 is coupled to the second housing portion 106. The first housing portion 112 may be coupled to the second housing portion 106 via laser welding, adhesives, mechanical fasteners, etc. In some examples, the first housing portion 112 defines a case, while the second housing portion 106 forms a portion of an overlay assembly, described further below, the overlay assembly further comprising a frame. The second housing portion 106 may comprise a transparent material, e.g., liquid silicone rubber (LSR). In some examples, an in-mold labeling (IML) layer is added for aesthetic reasons.

The second housing portion 106 includes a user interface. In this example, the user interface includes an action button 104 and a light emitting element 114, such as a light emitting diode (LED). The user interface may be devoid of a display, which enables a reduction in size and cost of the fluid infusion device 100. A status button 102 enables the user to press the status button 102 to turn on one or more lights indicative of a pump status. The action button 104 enables the user to clear alarms or alerts generated by the fluid infusion device 100 and to pair the fluid infusion device 100 with a remote device or portable electronic device associated with the user, such as the user's smart phone, tablet, smart watch, computer, continuous glucose monitor, etc. In some examples, the user may select both buttons simultaneously to initiate a software restart or a full fluid infusion device 100 shut down. In this example, the light emitting element 114 surrounds the status button 102, however, the light emitting element 114 may be positioned at other locations on the second housing 106. The light emitting element 114 may be integrated with the status button 102, or may be coupled to the status button 102 through any suitable technique, such as press-fitting, adhesives, in-mold electronics, light guides, etc. In addition, in certain examples, the status button 102 may comprise a metal dome to provide tactile feedback. In other examples, the status button 102 may be a cosmetic surface coupled to a force sensitive resistor (FSR) or a pressure sensor with a linear resonant actuator (LRA) that is programmed to vibrate and simulate the effect of button presses to provide tactile feedback. The light emitting element 114 provides a visual indicator of a status associated with the fluid infusion device 100. For example, the light emitting element 114 may comprise a multicolor LED, which is controlled to illuminate in different colors based on a status of the fluid infusion device 100. For example, the light emitting element 114 may be illuminated in green when the fluid infusion device 100 is operating properly, may be illuminated in red when there is an alarm or alert associated with the fluid infusion device 100, may be illuminated in blue when pairing the fluid infusion device 100 with the user's portable electronic device, etc. In some examples, the action button 104 may also include a light emitting element similar to the light emitting element 114.

The fluid infusion device 100 may additionally comprise a power supply (not shown). The power supply is any suitable device for supplying the fluid infusion device 100 with power, including, but not limited to, a battery. In some examples, the power supply is a rechargeable battery, which is fixed within the housing 108. The power supply may comprise a planar rectangular battery or a planar cylindrical battery. In such examples, the power supply is rechargeable via USB, wireless charging, etc. In the example of wireless charging, a charging coil of the fluid infusion device 100 may be configured such that the fluid infusion device 100 may be set down onto a charging pad for general charging or may be set into a form-fitting wireless charging receptacle with predetermined positioning. This charging receptacle may, itself, be battery powered and can slip over the fluid infusion device 100 for charging while on the go such that the fluid infusion device 100 remains functional during charging. Generally, it may be desirable that the power supply is chargeable for at least a 7-day use.

Within the housing 108, the fluid infusion device 100 includes processing circuitry (e.g., at least one processor) and a computer readable storage device or media, which are mounted to a printed circuit board (PCB) (not shown). The processing circuitry can be any custom made or commercially available processor, a central processing unit (CPU), a graphics processing unit (GPU), an auxiliary processor among several processors, a semiconductor based microprocessor (in the form of a microchip or chip set), a macroprocessor, any combination thereof, or generally any device for executing instructions. In certain examples, the fluid infusion device 100 includes more than one processor. The computer readable storage device or media may include volatile and nonvolatile storage in read-only memory (ROM), random-access memory (RAM), and keep-alive memory (KAM), for example. KAM is a persistent or nonvolatile memory that may be used to store various operating variables while the processor is powered down. The computer-readable storage device or media may be implemented using any of a number of known memory devices such as PROMs (programmable read-only memory), EPROMs (electrically PROM), EEPROMs (electrically erasable PROM), flash memory, or any other electrical, magnetic, and/or optical memory devices capable of storing data, some of which represent executable instructions, used by the processing circuitry in controlling components associated with the fluid infusion device 100.

The instructions may include one or more separate programs, each of which comprises an ordered listing of executable instructions for implementing logical functions. The instructions, when executed by the processor, may receive and process input signals; perform logic, calculations, methods and/or algorithms for controlling the components of the fluid infusion device 100; and generate signals to components of the fluid infusion device 100 to control the drive system and/or the light emitting element 114 based on the logic, calculations, methods, and/or algorithms.

The fluid infusion device 100 can include any number of hardware components that communicate over any suitable communication medium or a combination of communication mediums and that cooperate to process the signals from the user interface comprising the status button 102 and the action button 104; process signals received from the portable electronic device, perform logic, calculations, methods, and/or algorithms; and/or generate control signals to control features of the fluid infusion device 100.

Additionally, or alternatively, the one or more instructions, when executed by the processor, may enable receiving and processing signals from the user interface comprising the status button 102 and the action button 104 to generate one or more control signals to clear an alarm or alert associated with the fluid infusion device 100.

In some examples, sensing circuitry of a wearable medical device, e.g., a continuous glucose monitor, associated with the fluid infusion device 100 senses interstitial glucose data and other physiological data, and the processor of the fluid infusion device 100 determines whether an alarm is needed based on the data. In other examples, the fluid infusion device 100 determines that an alert is needed due to system issues, e.g., low battery, device pairing issues, pump failure, or high temperature.

A speaker within the housing 108 of the fluid infusion device 100 is responsive to one or more signals from the processor to generate a sound indicative of an alert. For instance, different alerts may be associated with different sounds (e.g., different frequencies, durations, tone patterns, etc.). The processor of the fluid infusion device 100 may determine the alert and cause the speaker to generate the sound associated with the alert. One example of the speaker is a piezoelectric speaker, but other examples of speakers are possible.

As described, in one or more examples, the fluid infusion device 100 may be fully enclosed, which can muffle the sound generated by the speaker. Moreover, the sound generated by the speaker can disperse within the fluid infusion device 100, lowering the volume of the sound.

In accordance with examples described in this disclosure, the fluid infusion device 100 may include a plurality of sound chambers in a stacked configuration (e.g., in the stacked configuration the sound propagates serially through the plurality of sound chambers). Each of the sound chambers may be separated by respective diaphragms of one or more diaphragms. The respective diaphragms may vibrate in response to the sound propagating through the fluid infusion device 100, which in turn causes the sound to propagate in a manner that preserves the volume of the sound (e.g., minimizes sound dispersion).

Furthermore, the housing 108 may comprise an overlay cover 110 that can also vibrate. The sound propagating through the fluid infusion device 100 causes the vibration of the housing 108 (e.g., the overlay cover 110) thereby providing an audible alert, alarm, or notification to the user. Because the overlay cover 110, which is a portion of the second housing 106 of the housing 108, is able to vibrate, the sound that propagates through the fluid infusion device 100 is audible by the user.

For instance, it may be possible for the speaker to generate a relatively high volume for the user to hear the sound. However, in such cases the speaker may consume more power than is desired. With the example techniques, the speaker can generate the sound at a reasonable volume, and the example techniques enable the sound to propagate in a manner where the sound is still audible to the user.

In some examples, a portion of the housing 108 (e.g., the overlay cover 110) is configured to vibrate for sound propagation purposes. The sound propagation system of stacked sound chambers is inside the housing 108 and may be aligned or nearly aligned with the overlay cover 110. Isolating vibration to a specific portion of the housing 108 may be advantageous to enable additional fortification of the housing 108. Additionally, isolating vibration abilities to the overlay cover 110 may be conducive to aesthetic design choices.

The action button 104 and the status button 102 may be sealed by a gasket such as an elastomeric, semi-solid, or similarly compliant material, including, but not limited to silicone, ethylene propylene diene terpolymer (EPDM), Polytetrafluoroethylene (PTFE), synthetic or natural rubbers, or fluoropolymer. Alternatively, sealing may be accomplished by means of a material exhibiting or comprising a high surface tension in combination with a gap between components that in combination do not allow the ingress of water or dust up to the levels anticipated with the fluid infusion device 100, for example, about 8 to about 12 feet for water and dust, such as that associated with an IP28 rating. The gasket may be compressed to form a hermetic seal between the housing 108 and the action button 104 and the status button 102. The hermetic seal inhibits the flow of fluids or other debris into the housing 108, which protects the internal components of the housing 108. Sealing associated with an IP28, rating may accomplish the anticipated ingress protection associated with the fluid infusion device 100. In some examples, dust ingress may not be tested, and IP ratings may include IPX6, IPX7, or IPX8. In other examples, the device is associated with a higher ingress protection rating, IP68, wherein the device is deemed dustproof.

The fluid infusion device 100 may function in any number of external environments, such as a home, an office, outdoors, etc. Due to the optimized size and shape and watertight and dustproof nature of the external housing, the fluid infusion device 100 can be used safely in a variety of environments.

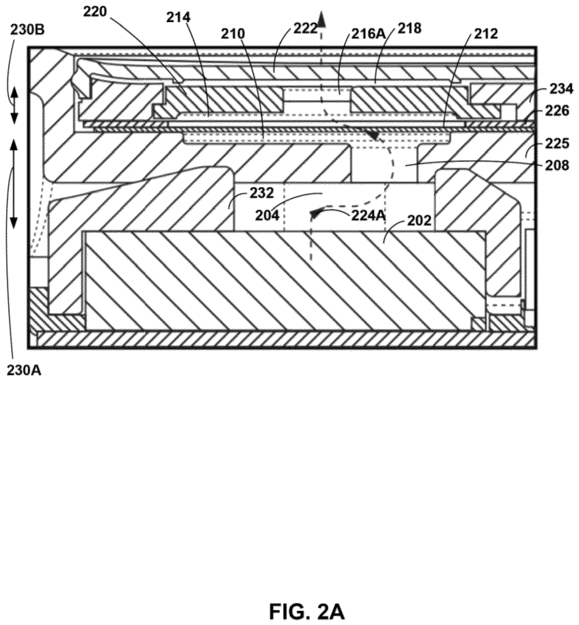

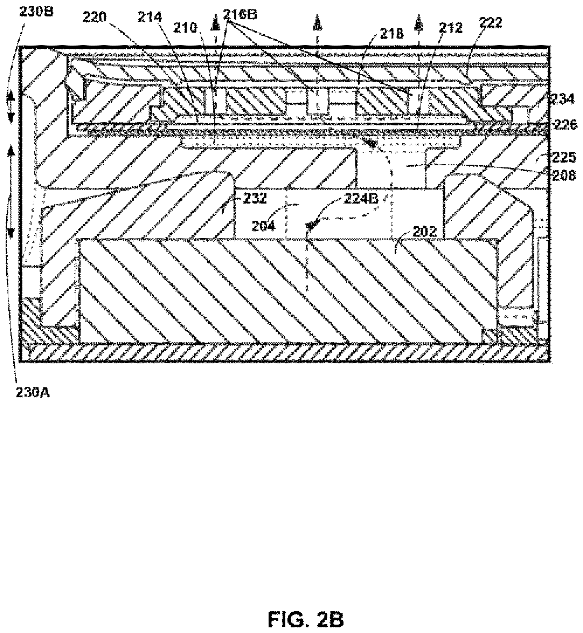

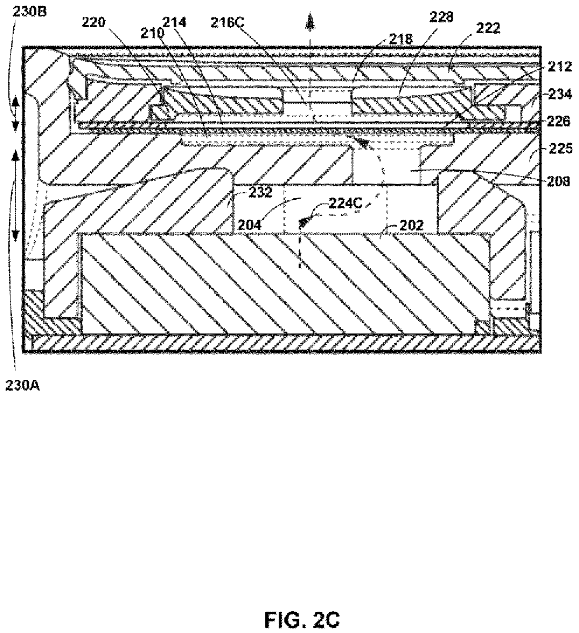

FIGS. 2A-2C are schematic diagrams illustrating cross-sectional views of exemplary configurations of stacked sound chambers in accordance with one or more techniques of this disclosure. FIGS. 2A-2C may be configured substantially similarly to one another, with differences between them discussed herein. For example, the differences comprise differences between a second sound tuning hole 216A (FIG. 2A), second sound tuning holes 216B (FIG. 2B), and second sound tuning hole 216C (FIG. 2C) (collectively, "second sound tuning hole(s) 216") and the resulting differences between the sound propagation pathways 224A (FIG. 2A), 224B (FIG. 2B), and 224C (FIG. 2C) (collectively, "sound propagation pathways 224). For instance, the dashed line in FIGS. 2A-2C illustrates an example of the sound propagation pathway identified as 224A-224C, respectively.

As described in more detail, in FIGS. 2A-2C illustrate a first sound chamber 230A and a second sound chamber 230B. For example, the first sound chamber 230A includes a first sound tuning hole 208. The first sound chamber 230A may also include constrained air volumes 204 and 210. The second sound chamber 230B includes the second sound tuning hole(s) 216. The second sound chamber 230B may also include constrained air volumes 214 and 218. In general, the smaller the volume of the constrained air volumes 210, 214, and 218, the more the pressure increases as each diaphragm vibrates and displaces a prescribed air volume. A relatively larger pressure increase is associated with greater sound pressure wave propagation. In some examples, the first air volume 204 is approximately 21.5 cubic mm. Other air volumes are also possible. For the purposes of this disclosure, the term "approximately" describes manufacturing tolerances, e.g., ±10%. As described in more detail, a first diaphragm 212 separates the first sound chamber 230A and the second sound chamber 230B. There may be additional sound chambers, similar to the first and second sound chambers 230A, 230B, that are stacked, with respective diaphragms separating the sound chambers.

For instance, in FIGS. 2A-2C, the second sound chamber 230B is stacked on the first sound chamber 230A such that in the stacked configuration the sound propagates serially through the plurality of sound chambers, shown with the sound propagation pathways 224. That is, the sound propagates from one sound chamber to the next in a serial manner.

With respect to FIGS 2A-2C, a speaker 202, e.g., a surface mounted piezoelectric, initiates sound propagation. Other examples of the speaker 202 are possible. A surface mounted piezoelectric speaker is described for ease of description only. In some examples, the speaker 202 may be attached to a PCB (printed circuit board). The PCB may additionally comprise the processing circuitry (e.g., processor) described above that outputs signals that cause vibration of the speaker. In the example of the fluid infusion device 100, the sound propagation may be initiated to create an alarm related to the operation of the fluid infusion device or to notify a user of a glucose level.

The sound resulting from vibration of the speaker 202 travels through a first air volume 204. The first air volume 204 is a constrained air volume enclosed by a volume constraining apparatus 232. In some examples, the volume constraining apparatus 232 may comprise rubber. The first air volume 204 provides a pathway from the speaker 202 to the first sound tuning hole 208. The first sound tuning hole 208 may be cylindrical and is sized to propagate sound at the dB level provided by the speaker 202. Different shapes may be possible. However, a cylinder may be suitable and cause desired sound propagation due to acoustic properties of circular openings. Generally, circular openings enable a greater uniformity of sound distribution to minimize the effects of diffraction and turbulence relative to other shapes. Additionally, circular openings are simple to manufacture and can be formed using standard tools and methods. The first sound tuning hole 208 is in contact with a second air volume 210. The second air volume 210 enables sound-causing vibrations to propagate from the first sound tuning hole 208 to the first diaphragm 212.

As illustrated, the first diaphragm 212 separates the respective sound chambers 230A and 230B in the stacked configuration. The first diaphragm 212 vibrates in response to the sound propagating from the first sound chamber 230A of the respective sound chambers to the second sound chamber 230B of the respective sound chambers.

The first diaphragm 212 is thermally bonded to a cover 225. The cover 225 may be substantially similar to or the same as the first housing portion 112. Methods other than thermal bonding may be used to attach the first diaphragm 212 to the cover 225. Thermal bonding may be more conducive to creating a chemical resistant and waterproof device than some other bonding methods. In some examples, the first diaphragm 212 comprises polyetherimide (PEI). Other materials, e.g., aluminum sheets, embossed aluminum sheets, paper laminates, polyester (PET), and polypropylene, are also possible, but PEI may be suitable to use because PEI maintains its ability to vibrate after thermal bonding, i.e., has a glass transition temperature above thermal bonding temperatures, even with minimal thickness (e.g., .001 in.). PEI can undergo cyclic loading and does not tend to exhibit fatigue or stress-relieving cracking under cyclic loading conditions. Additionally, PEI is associated with a high tear strength. Additionally, PEI is a transparent, uncolored material. In the example of the fluid infusion device 100, one or more LEDs (e.g., the light emitting element 114) may be used to provide indications to a user. Because PEI is transparent, light is able to travel through the PEI, enabling the use of LEDs. The fluid infusion device 100 serves merely as an example. Many medical devices use LEDs and other lights to provide notifications to users.

The first diaphragm 212 may have a diameter of about 8 millimeters (mm). The diameter of the first diaphragm 212 may vary from 4 mm to 12 mm. Smaller and more aesthetically pleasing devices may improve user experience. In the example of the fluid infusion device 100, the user may have to wear the fluid infusion device 100 at all times. Identifying a minimum diaphragm size results in a smaller overall device, making the fluid infusion device 100 easier for a user to wear and potentially increasing user comfort and compliance. An 8 mm diameter is approaching a minimum diameter to successfully enable sound propagation via vibration of the first diaphragm 212. Larger diaphragm diameters are also possible, and in some applications, a larger diameter may be desirable.

The first diaphragm 212 may have a thickness of .001 (in.). The thickness of the first diaphragm 212 may vary from approximately 0.0005 in. to 0.005 in. The thickness of the diaphragm may be based in part on the properties of the selected material. In the example of PEI and at the frequency desired for alert output of this example, the diaphragm must be sufficiently thin to enable vibration but must also be thick enough to resist tearing or other destruction caused by cyclic vibration. If a material other than PEI is selected for the first diaphragm, a different thickness range may apply due to differences in material properties. As an example, PEI is relatively flexible, enabling the PEI layer to be thicker than some other potential materials while still being able to vibrate. Other materials may be less flexible and may therefore need to be thinner to enable vibration. In some cases, multiple layer thicknesses may need to be tested to identify an optimal thickness of the first diaphragm for a selected material.

The first diaphragm 212 is in contact with a third air volume 214. The third air volume 214 and the second air volume 210 enable the first diaphragm 212 to vibrate and provide a pathway for the vibrations generated in the first diaphragm 212 to travel through a second sound tuning hole(s) 216. The second sound tuning hole(s) 216 are constrained by a sound spacer 220 and comprise one or more through-holes in the sound spacer 220. The sound spacer 220 is connected to the first diaphragm 212 by an adhesive 226, e.g., a double-sided foam tape. The adhesive 226 adheres an overlay assembly comprising the sound spacer 220, a frame 234, and an exterior cover 222 to the first diaphragm 212. The exterior cover 222 may be the same as the second housing portion 106. Other adhesive types are also possible and may function equally well. Vibrations propagating through the second sound tuning hole(s) 216 via a fourth air volume 218 cause vibration of the exterior cover 222. The frame 234 provides the primary structure of the overlay assembly. In some examples, the frame 234 is composed of an opaque polyamide, e.g., nylon. With respect to FIGS. 2A-2C, frame 234 contains through-holes for the status button 102, the action button 104, an arrow icon, and the sound spacer 220. In such examples, the sound spacer 220 is press fit into a respective through-hole of the frame 234. The sound spacer 220 and the respective through-hole of the frame 234 are keyed to prevent rotation, thereby immobilizing the sound spacer 220 with respect to the frame 234. The sound spacer 220 is installed into the frame 234 until it reaches a positive stop on an interior surface of the frame 234. The sound spacer 220 may be formed with an opaque polyamide, e.g., nylon.

In some examples, the geometry of the frame 234 takes forms other than the form depicted in FIGS. 2A-2C. For example, the frame 234 may comprise one or more air channels or chambers to create constrained air volumes within the fluid infusion device 100. As an example, the frame 234 may include an air channel between the sound spacer 220 and the action button 104 to form a constrained air volume. There may be wiring and other components within the constrained air volume, but the action button 104 may be formed with sufficiently flexible material to allow the action button 104 to vibrate and propagate the sound. As another example, the frame 234 may comprise a plurality of shallow chambers with acoustic reflectors to focus sound pressure toward the action button 104 and the status button 102. The action button 104 and the status button 102 may vibrate freely like a diaphragm in response to sound pressure generated by the speaker 202.

In other examples, the frame 234 includes a plurality of small through-holes to enable sound propagation instead of or in addition to the through-hole for the sound spacer 220. The small through holes are covered by the exterior cover 222. In some examples, the small through-holes are not visible through the exterior cover 222. As another example, the frame 234 comprises a plurality of separate mobile elements and a main body. The mobile elements can move with respect to the main body and enable vibration of the exterior cover 222 in response to sound pressure generated by the speaker 202. In other examples, the frame 234 may be configured such that the status button 102 and/or the action button 104 can vibrate like a diaphragm. Walls of the frame 234 around the status button 102 and/or the action button 104 may be removed. In other examples, the rear surface of the frame 234 may have shallow chambers that enable the first diaphragm 212 to vibrate freely, wherein only the perimeter of the frame 234 is adhered to the overlay assembly. The frame 234 may or may not have support ribs. In some examples, the exterior cover 222 comprises LSR. In other examples, different materials may be used. LSR can be tailored to resist various chemicals, which may protect the fluid infusion device 100 from any damage. In the example of the fluid infusion device 100, the fluids may include chemicals capable of damaging some materials in the event of a leak. Additionally, the fluid infusion device 100 may be subjected to substances and chemicals in an external environment, such as sunscreen, bug spray, hand lotions, etc. The chemical resistance of LSR may be advantageous in protecting the fluid infusion device 100 from various environments. LSR can also vary in hardness, and hardness of LSR can be optimized to enable button pressing while still having a high enough tear strength to prevent tearing, e.g., tearing inadvertently caused by the user. Additionally, LSR can undergo cyclic loading and does not exhibit fatigue or stress-relieving cracking under loading conditions.

Additionally, LSR is transparent, which may be advantageous in devices with user interfaces that include LEDs or other lighting systems. As described above, in some examples, the exterior cover 222 includes the overlay cover 110, and the overlay cover 110 is a separate material than the exterior cover 222, where the overlay cover 110 forms a portion of the exterior cover 222. In other examples, the exterior cover 222 and the overlay cover 110 are formed as the same material, in which case the exterior cover 222 and the overlay cover 110 become one cover.

Accordingly, in some examples, a portion, e.g., the overlay cover 110, of the exterior cover 222 serves as a second type of diaphragm. In other examples, the entire exterior cover 222 or the entire housing 108 (FIG. 1) serves as the second type of diaphragm. In this way, the exterior cover 222 may be part of an external housing 108 that fully encloses the fluid infusion device 100 and includes the overlay cover 110. The overlay cover 110 may be configured to vibrate in response to the sound propagating through the plurality of the sound chambers 230A, 230B in the stacked configuration. The exterior cover 222, including the overlay cover 110, may be formed with LSR.

In some examples, e.g., examples in which all of the exterior cover 222 is configured to vibrate, the exterior cover 222 varies in thickness. For example, the exterior cover 222 may comprise a relatively thick layer of material, e.g., LSR, over the action button 104 and the status button 102 and around the perimeter of the exterior cover 222. The relatively thick layer of the exterior cover 222 over the action button 104, the status button 102 may maintain desirable tear prevention qualities of the exterior cover 222, and the relatively thin layer elsewhere (e.g., over the stacked sound chambers 230A, 230B) may reduce any sound dampening associated with the layer of the exterior cover 222.

In some examples, it may be desirable for some portions, i.e., portions other than the overlay cover 110, the action button 104, and the status button 102 (FIG. 1), of the exterior cover 222 to be opaque rather than transparent for aesthetic reasons. One method of making the exterior of the fluid infusion device 100 appear opaque involves applying an IML layer, i.e., a polyethylene terephthalate (PET) sheet coated in ink to the exterior cover 222, e.g., to make the majority of the device appear opaque but enable light to pass through certain areas, e.g., LEDs, buttons. Adding an IML layer can result in an aesthetically pleasing, uniform device. The IML layer may be printed with graphic inks, which may be opaque in regions not intended to enable light to pass and semi-translucent in areas to be illuminated. The frame 234 may be overmolded via plastic injection molding onto the printed ink surface of the IML layer, and concurrently, the opposite side of the IML layer may be thermoformed into a desired geometry. The exterior cover 222 may then be overmolded via liquid injection molding onto the opposite, i.e., non-printed surface, of the IML layer.

The addition of an IML to the exterior cover 222 may limit the ability of the exterior cover 222 to vibrate. In some examples, the IML is not applied to an 8 mm diameter circular portion of the exterior cover 222, i.e., the overlay cover 110, to maintain the vibrating abilities of the overlay cover 110.

With respect to FIG. 2A, a sound propagation pathway 224A indicates a possible route of sound propagation that results from the example described in FIG. 2A. The sound pathway 224A moves through the sound tuning hole 216A to cause vibration of the exterior cover 222 that generates sound.

In some examples, the sound tuning hole(s) 216 may be slightly offset, as depicted in the examples of FIG. 2A-2C. In other examples, the sound tuning holes may not be offset, i.e., the first sound tuning hole 208 may be aligned with the second sound tuning hole 216A, i.e., axes of the first sound tuning hole 208 and the second sound tuning hole 216A may be aligned. The arrangement of the sound tuning holes may depend on spacing constraints. As an example, the sound tuning holes may be offset to provide space for a keypad or another portion of the device within the housing 108.

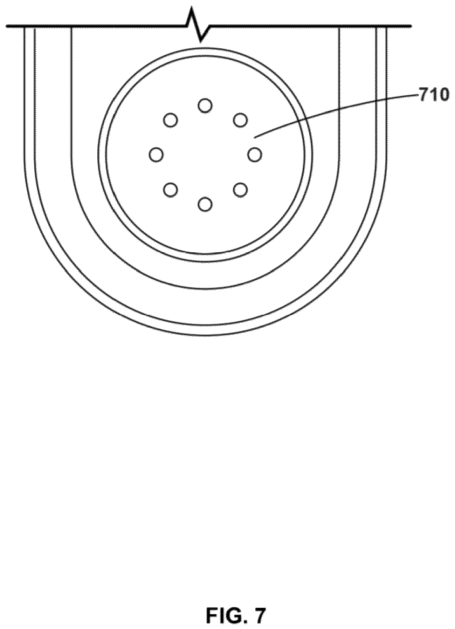

With respect to FIG. 2B, a sound propagation pathway 224B indicates a possible route of sound propagation associated with the example depicted in FIG. 2B. At the third air volume 214, sound propagation pathway splits into multiple directions associated with a plurality of the second sound tuning holes 216B. The second sound tuning holes 216B comprise a plurality of through-holes in the sound spacer 220. In some examples, a plurality of relatively smaller sound tuning holes is advantageous, both to prevent tearing of the exterior cover 222 and for aesthetic reasons. The exterior cover 222 may be prone to tearing due to the minimal thickness required for vibration. In some examples, the exterior cover 222 has a nominal thickness of 0.4 mm. Additionally, in some examples, the exterior cover 222 is clear at the overlay cover 110, the action button 104, and the status button 102 (FIG. 1), making the sound spacer 220 visible to the user. The clear overlay cover 110 therefore enables additional graphical and aesthetic options. In some examples, the second sound tuning hole(s) 216 in the sound spacer 220 may be patterned to emulate the appearance of a speaker (FIG. 7).

With respect to FIG. 2C, a sound propagation pathway 224C indicates a possible route of sound propagation associated with the example depicted in FIG. 2C. The second sound tuning hole 216C ends in a curve 228, resulting in a concave sound spacer 220. The curve 228 may reduce stress concentration by removing any sharp edges associate with other forms of the second sound tuning hole(s) 216. The second sound turning hole 216C may be advantageous to prevent tearing caused by cyclic pressing of a second diaphragm, i.e., the overlay cover 110, of the exterior cover 222 by a user. Additionally or alternatively, the exterior cover 222 comprises a curved geometry to likewise prevent tearing.

Accordingly, FIGS. 2A-2C illustrate examples of a medical device (e.g., the fluid infusion device 100) that includes the speaker 202 comprising a vibrating membrane and configured to generate a sound that propagates through the medical device. As an example, the speaker 202 is a surface-mounted piezoelectric. The medical device also includes a plurality of the sound chambers 230A, 230B in a stacked configuration and configured to propagate the sound generated by the speaker 202 through the one or more sound tuning holes 208, 216 of the sound chambers 230A, 230B. In the stacked configuration the sound propagates serially through the plurality of the sound chambers 230A, 230B.

One or more diaphragms (e.g., the first diaphragm 212), within the medical device, may be configured to vibrate in response to the sound propagating though the medical device. For instance, each of the one or more diaphragms separates the respective sound chambers 230A, 230B in the stacked configuration and vibrates in response to the sound propagating from a first sound chamber (e.g., the sound chamber 230A) of the respective sound chambers to a second sound chamber (e.g., the sound chamber 230B) of the respective sound chambers.

In one or more examples, a material of at least one, including some or all, of the one or more diaphragms may be PEI. At least one of the one or more diaphragms may be circular (e.g., have a diameter of 8 millimeters). In some examples, at least one of the one or more diaphragms has a thickness of 0.0005 in. to 0.005 in

Although two sound chambers 230A, 230B are illustrated, there may be additional sound chambers, similar to the sound chambers 230A, 230B that are stacked. For instance, as illustrated, the sound propagation pathways 224 illustrate sound serially propagating through the sound chambers 230A, 230B. In examples where there are additional sound chambers, the sound propagation pathways 224 would flow through those additional sound chambers. Also, respective diaphragms, such as the diaphragm 212, may separate those additional sound chambers, and vibrate in response to the sound propagating through the respective sound chambers that the respective diaphragms separate.

Furthermore, there may be constrained volumes of air that form part of the respective sound chambers 230A, 230B so that the respective diaphragms 212 can vibrate. That is, each of the plurality of sound chambers 230A, 230B may include respective constrained air volumes. Each of the respective diaphragms (e.g., like the diaphragm 212) may be configured to vibrate within the respective constrained air volumes.

For example, the first sound tuning hole 208 is separated from the speaker 202 by the first air volume 204. The second sound tuning hole(s) 216 is separated from the first sound tuning hole 208 by the second air volume 210, the first diaphragm 212, and the third air volume 214, the first diaphragm 212 being in contact with the second air volume 210 and the third air volume 214 and configured to vibrate, and the second sound tuning hole(s) 216A is separated from a second diaphragm by the fourth air volume 218, the fourth air volume being in contact with the second diaphragm, and wherein the second diaphragm comprises the overlay cover 110. In one or more examples, the sound tuning hole 208 and/or the sound tuning hole(s) 216 may be in a circular configuration.

An external housing 108 may fully enclose the medical device, which includes the second housing portion 106. The second housing portion 106 may include an overlay cover 110 that is formed with LSR, but other materials are possible. In some examples, the entirety of the second housing portion 106 is formed with LSR. At least a portion of the second housing portion 106 (e.g., the overlay cover 110) may be configured to vibrate in response to the sound propagating through the plurality of the sound chambers 230A, 230B in the stacked configuration.

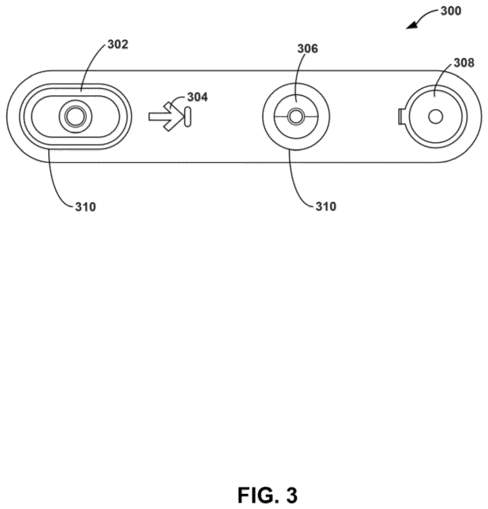

FIG. 3 is a bottom view of an exemplary external housing portion 300 configured for user interaction, in accordance with one or more techniques of this disclosure. The housing portion 300 is an example of the second housing portion 106 (FIG. 1) and comprises spaces for a status button 302, an arrow icon 304, an action button 306, and a sound spacer 308. In some examples, the status button 302, the arrow icon 304, the action button 306, and the sound spacer 308 are all covered by the exterior cover 222 (FIGS. 2A-2C), and an IML layer surrounds them, causing the fluid infusion device 100 to appear uniform in color.

The sound spacer 308 may be press fit and keyed into the frame 234 supporting the external housing portion 300 to prevent rotation. The sound spacer 308 may be installed into the housing 108 (e.g., the second housing portion 106) until the sound spacer 308 reaches a positive stop on the interior surface of the second housing portion 106. In one or more examples, the sound spacer 308 is composed of an opaque polyamide (nylon). The front surface of the sound spacer 308 may include graphics to indicate that the sound spacer 308 does not represent a button, but other techniques to indicate that the sound spacer 308 is not a button are possible. In some examples, the graphics may be created via in-mold labeling by printing them on a clear polyester graphic sheet and overmolding the graphics with the sound spacer material of the sound spacer 308. Alternatively, the graphics are added to the front surface of the sound spacer 220 by pad printing, transfer printing, or laser marking.

The status button 302 and the action button 306 may have respective through-holes in the frame 234 to enable forces associated with button presses to interact with a keypad located below the frame 234. In some examples, the status button 302 and the action button 306 may be sealed using respective ones of the gasket 310. Examples of gasket 310 include an elastomeric, semi-solid, or similarly compliant material, including, but not limited to silicone, ethylene propylene diene terpolymer (EPDM), Polytetrafluoroethylene (PTFE), synthetic or natural rubbers, or fluoropolymer. The arrow icon 304 may provide indications to the user related to alerts or other device operations. The arrow icon 304, the status button 302, and action button 306 may optionally include light emitting elements such as LEDs to provide further guidance or indications to users. In some examples, the status button 302 may enable the user to check a status of the fluid infusion device 100. For example, the user may press the status button 302 to cause lights indicative of the fluid infusion device 100 status. The action button 306 may enable the user to pair the fluid infusion device 100 to another device, such as the user's smart phone, tablet, smart watch, computer, continuous glucose monitor, etc. The status button 302 and the action button 306 may additionally enable the user to restart the fluid infusion device 100 software. Other purposes for the status button 302 and the action button 306 are also possible. The exterior cover 222 (FIGS. 2A-2C) are generally compressible to enable button presses by users. Some users may have arthritis or other conditions that limit fine motor skills and/or strength, so it may be advantageous for the status button 302 and the action button 306 should be sufficiently large and pliable to enable such users to interact with the device easily.

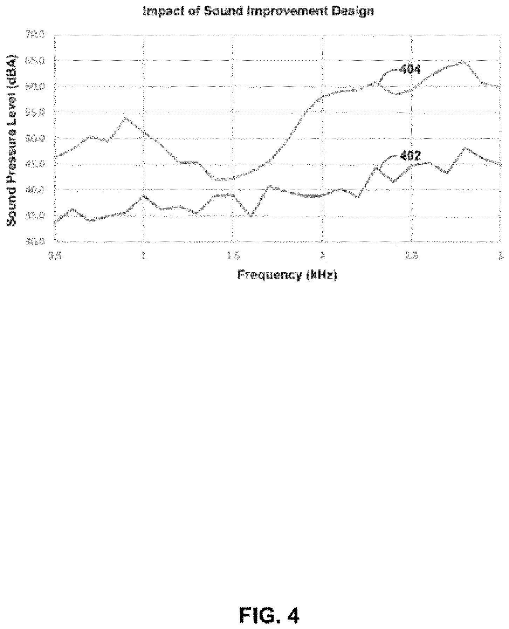

FIG. 4 is a graphical illustration of a relationship between frequency and sound pressure level, in accordance with one or more techniques of this disclosure. Line 402 represents output sound pressure levels (dBA) in an example of a fluid infusion device that was not optimized for sound output using the example techniques described in this disclosure. Specifically, the example of the fluid infusion device represented by the line 402 comprises the speaker 202 within the housing 108 and does not include stacked sound chambers. After optimization of sealing adhesive geometry, sound spacer geometry, material selection, and volume constraining apparatus, sound output increased at all tested frequencies, as indicated by the line 404. Line 404 is representative of the sound outputs associated with either of stacked sound chamber configurations FIGS. 2A or 2B. For purposes of generating alerts, it may be advantageous to ensure that desired sound pressure levels are achieved and consistent between 2 kilohertz (kHz) and 3 kHz. In some applications, a minimum sound pressure may be based on standard requirements, such as FDA requirements, e.g., IEC standard 60601-2-42. As an example, a minimum sound pressure of 45 dB may be required for high priority alarms. Using the methods of this disclosure, sound pressures between 55 and 65 dBA were consistently achieved between 2 kHz and 3 kHz.

In other examples, different requirements regarding sound pressure output and frequency may be in place. To adjust performance of the fluid infusion device 100's sound propagation system to fit different requirements, the sound tuning holes 208 and 216 can be adjusted as needed, as well as the volume of the constrained air volumes 204, 210, and 214.

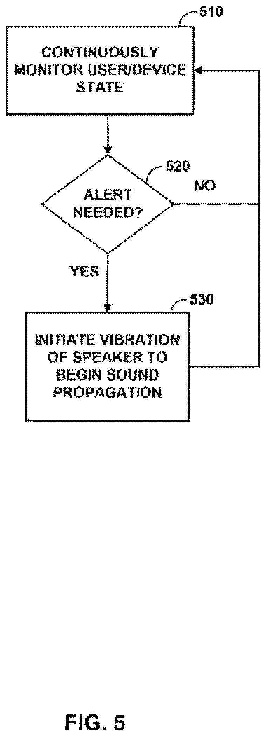

FIG. 5 is a flow diagram illustrating an exemplary operation for initiating sound propagation, in accordance with one or more techniques of this disclosure. The fluid infusion device 100 may continuously monitor user and/or device state (510). In some examples, a separate monitoring device, e.g., a continuous glucose monitor, continuously monitors interstitial glucose levels. The two medical devices comprise a medical device system in communication with one another. The medical devices may communicate via one or more of near-field communication (NFC), radio frequency (RF), Bluetooth, etc. The user state can be related to any one or more physiological conditions of a user, e.g., interstitial glucose level, SPO₂, blood pressure, respiration rate, etc.

If the user and/or device is experiencing a state, e.g., low glucose level, low battery, pump failure, high temperatures, or pairing issues, that satisfies a condition for an alarm (YES of 520), the device system initiates vibration of a speaker, e.g., the speaker 202, to begin sound propagation and continues to monitor user and/or device state. In some examples, in addition to alerting the user via initiating sound propagation, processing circuitry of the fluid infusion device 100 may illuminate the light emitting element 114 or other light emitting elements, cause vibration of a linear resonant actuator (LRA) within the fluid infusion device 100, or control communication circuitry to communicate with another device, e.g., the user's smartphone, to initiate smartphone annunciation schemes. In examples in which a medical device system monitors physiological data, the continuous glucose monitor of the device system may include a processor configured to determine whether data collected is indicative of a state requiring an alert. Communication circuitry of the continuous glucose monitor may then send a communication to the fluid infusion device 100. Additionally or alternatively, the communication circuitry of the continuous glucose monitor may transmit data, e.g., interstitial glucose data, to the fluid infusion device the 100 for processing, and the fluid infusion device 100 determines whether an alert is needed.

Additionally or alternatively, the fluid infusion device 100 monitors device state. Examples of devices states that may cause the fluid infusion device 100 to generate an alert include, but are not limited to: low battery, pump failure, high temperatures, and pairing issues.

In the example of a low battery alert, the processor of the fluid infusion device 100 may determine that the battery level is low and needs to be charged to prevent a lapse in insulin administration. The low battery level alert may be generated when the battery level drops below a threshold value, e.g., 5%, or when the estimated battery life drops below a threshold, e.g., 8 hours. The alert may advise the user to charge the device.

In the example of a pump failure alert, the processor of the fluid infusion device 100 may determine that the pump has failed. As an example, a determination of pump failure may be associated with an occlusion of the pump. A plurality of pump issues can cause pump occlusion. The processor of the fluid infusion device 100 may determine pump occlusion by analyzing the force required to pump a certain volume of insulin via a motor of the fluid infusion device 100. In a working fluid infusion device 100, the motor may generate a certain force to pump a volume of insulin. If more force than the expected force is required to pump the volume of insulin, the processor may determine there is a pump occlusion. In some examples, the pump is occluded to such an extent that insulin is no longer being provided to a user via the fluid infusion device 100. In this case, the processor may determine that alerts for both the device and low glucose levels are needed.

In the example of a high temperature alert, the processor of the fluid infusion device 100 may determine that the fluid infusion device 100 is being exposed to high temperatures that could decrease device performance and/or render the insulin within the pump ineffective. The high temperature could be caused by the external environment, e.g., placing the device in direct sunlight, or due to charging or some other action that may cause overheating. The fluid infusion device 100 may include a temperature sensor configured to transmit data to the processor. The processor may determine the device is overheating by comparing the temperature sensor data to a threshold value over a period of time, e.g., device has been above threshold for >5 minutes. The processor determines an alert is needed to advise the user to take an action to cool the device, e.g., unplug the fluid infusion device 100 or move to a cooler area.

In the example of pairing issues, the fluid infusion device 100 may be in communication with a smartphone or other computing device of the user. Since the fluid infusion device 100 does not include a display, some notifications or details regarding alerts may be transmitted to the smartphone of the user. The fluid infusion device 100 may communicate with the smartphone via one or more of near-field communication (NFC), radio frequency (RF), far-field communication, Bluetooth, or any other communication method. In the example of the fluid infusion device 100 communicating with the smartphone via Bluetooth, the fluid infusion device 100 and the smartphone may occasionally disconnect. The processor of the fluid infusion device 100 may determine that a disconnection occurred and may determine an alert is needed to inform the user of the disconnection.

Based on the one or more determinations that an alert is needed, the processor of the fluid infusion device 100 initiates vibration of the speaker 202 (FIGS. 2A-2C) to begin sound propagation (530). If the user and/or device is not experiencing a user and/or device state that necessitates an alarm (NO of 520), the device system continues to monitor user and/or device state.

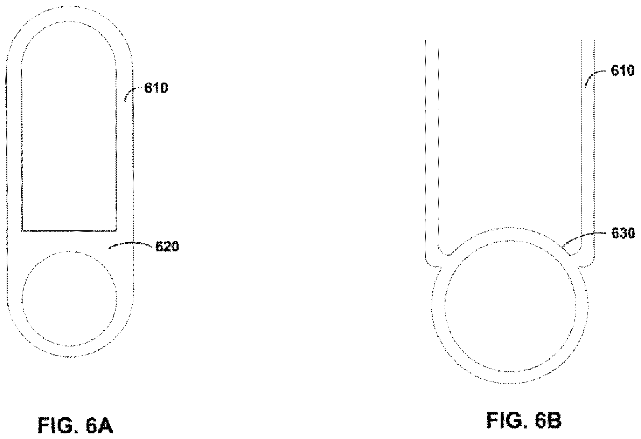

FIGS. 6A and 6B are diagrams depicting exemplary geometries of a thermal bonding adhesive, in accordance with one or more techniques of this disclosure. In some examples, the first diaphragm 212 is a portion of a sealing sheet applied on top of the cover 225 to seal any circuitry within the device, as well as the sound propagation system from external air. The thermal bonding adhesive is applied to the cover and sealing sheet and is shaped to maintain the circular shape of the first diaphragm 212, essentially forming an edge mounted diaphragm. In some examples, the thermal bonding adhesive is tacked to the first diaphragm 212 via a relatively low temperature lamination process, resulting in an assembly of the thermal bonding adhesive and the first diaphragm 212. The assembly may then be placed onto the cover 225 and bonded via a relatively high temperature and pressure.

With respect to FIG. 6A, a thermal bonding adhesive 610 is shaped to seal the perimeter of the sealing sheet to the cover 225. A bridge 620 creates a circular opening such that the thermal bonding adhesive does not cover the part of the diaphragm that vibrates, and only comes into contact with that portion of the diaphragm that is adhered to the device. FIG. 6B is substantially similar to FIG. 6A, with the differences described herein. FIG. 6B provides a zoomed in view of the thermal bonding adhesive 610. FIG. 6B includes an arched bridge 630. Depending on the adhesive selected, different bonding adhesive geometries may be desirable. In some examples, bonding adhesive geometries with greater surface areas may be associated with more robust adhesion. Additionally, the thermal bonding adhesive geometry can be optimized to ensure reproducibility in manufacturing, i.e., geometry can be optimized to ensure the adhesive behaves predictably during assembly.

FIG. 7 depicts an exemplary second sound tuning hole configuration. FIG. 7 may be a top view of FIG. 2B, wherein a plurality of second sound tuning holes 216B comprise a plurality of through-holes in the sound spacer 220. The plurality of second sound tuning holes 216B can be patterned in a circular configuration 710. The circular configuration 710 is visible through the exterior cover 222 (FIGS. 2). The circular configuration 710 may prevent unintended user interaction, e.g., pressing on the speaker portion, by making it clear that the speaker portion is not a button. Other patterns are also possible to use and may likewise serve to prevent the user from assuming the speaker portion is a button.

Various examples have been described. These and other examples are within the scope of the following claims.

Further disclosed herein is the subject-matter of the following clauses:
1. A medical device comprising:
   a speaker comprising a vibrating membrane and configured to generate a sound that propagates through the medical device;
   a plurality of sound chambers in a stacked configuration and configured to propagate the sound generated by the speaker through one or more sound tuning holes of the sound chambers, wherein in the stacked configuration the sound propagates serially through the plurality of sound chambers;
   one or more diaphragms, within the medical device, configured to vibrate in response to the sound propagating though the medical device, wherein each of the one or more diaphragms separates respective sound chambers in the stacked configuration and vibrates in response to the sound propagating from a first sound chamber of the respective sound chambers to a second sound chamber of the respective sound chambers;
   an external housing fully enclosing the medical device and comprising an overlay cover, wherein the overlay cover is configured to vibrate in response to the sound propagating through the plurality of sound chambers in the stacked configuration.
2. The medical device of clause 1, wherein at least a first sound tuning hole is separated from the speaker by a first air volume, wherein the first air volume is adjacent to the speaker and initiates a sound propagation pathway, a second sound tuning hole is separated from the first sound tuning hole by a second air volume, a first diaphragm and a third air volume, the first diaphragm being in contact with the second air volume and the third air volume and configured to vibrate, and the second sound tuning hole is separated from a second diaphragm by a fourth air volume, the fourth air volume being in contact with the second diaphragm, and wherein the second diaphragm comprises the overlay cover.
3. The medical device of any of clauses 1 or 2, wherein each of the plurality of sound chambers includes respective constrained air volumes, and wherein each of the respective diaphragms is configured to vibrate within the respective constrained air volumes.
4. The medical device of any of clauses 1-3, further comprising:
   processing circuitry configured to:
   determine that an audible alert is to be output; and
   upon determining that the audible alert is to be output, cause the speaker to generate the sound that propagates through the medical device through the plurality of sound chambers in the stacked configuration.
5. The medical device of any of clauses 1-4, wherein the speaker is a surface-mounted piezoelectric device.
6. The medical device of any of clauses 1-5, wherein a material of at least one of the one or more diaphragms is polyetherimide (PEI) or liquid silicone rubber (LSR).
7. The medical device of any of clauses 1-6, wherein a material of the overlay cover is liquid silicone rubber (LSR).
8. The medical device of any of clauses 1-7, wherein at least one of the one or more diaphragms is circular.
9. The medical device of clause 8 or of any of clauses 1-8, wherein at least one of the one or more diaphragms has a diameter between approximately 4 millimeters to 12 millimeters.
10. The medical device of any of clauses 1-9, wherein at least one of a one or more diaphragms has a thickness between 0.0005 inches to 0.005 inches.
11. The medical device of any of clauses 1-10, wherein the medical device is a handheld insulin pump device.
12. The medical device of any of clauses 1-11, wherein at least one of the plurality of sound chambers in the stacked configuration comprises a plurality of the sound tuning holes in a circular configuration.
13. The medical device of any of clauses 1-12, wherein the first diaphragm fully seals the medical device to achieve a watertight medical device.
14. The medical device of any of clauses 1-13, wherein the medical device has an ingress protection rating of IP28, IP38, IP48, IP58, IP68, IPX8, IPX7, IPX6, or IPX5.
15. The medical device of any of clauses 1-14, wherein a thermal bonding adhesive is applied to seal the first diaphragm to the external housing, and wherein the thermal bonding adhesive geometry includes an arched bridge.
16. The medical device of any of clauses 1-15, wherein the sound tuning holes are axially misaligned.
17. A method of generating an audible alert comprising:
   determining that an audible alert is to be output; and
   upon determining that the audible alert is to be output, causing a speaker comprising a vibrating membrane to generate a sound that propagates through the medical device through a plurality of sound chambers in a stacked configuration and configured to propagate the sound generated by the speaker through one or more sound tuning holes of the sound chambers, wherein in the stacked configuration the sound propagates serially through the plurality of sound chambers, wherein the sound chambers in the stacked configuration are separated by one of one or more diaphragms and each of the one or more diaphragms vibrates in response to the sound propagating from a first sound chamber of the respective sound chambers to a second sound chamber of the respective sound chambers, wherein an external housing fully encloses the medical device and comprises an overlay cover, wherein the overlay cover is configured to vibrate in response to the sound propagating through the plurality of sound chambers in the stacked configuration.
18. The method of clause 17, wherein the medical device comprises the features of any of clauses 2-16 or of any of clauses 1-16.
19. A method of manufacturing a medical device, the method comprising:
   forming a first sound chamber of a plurality of sound chambers in a stacked configuration, wherein the plurality of sound chambers is configured to propagate a sound generated by a speaker through one or more sound tuning holes of sound chambers, wherein in the stacked configuration the sound propagates serially through the plurality of sound chambers;
   forming a first of a plurality of diaphragms within the medical device;
   forming a second sound chamber of the plurality of sound chambers;
   wherein each of the plurality of diaphragms is configured to vibrate in response to the sound propagating though the respective sound chambers and one of the one or more diaphragms separates respective sound chambers in the stacked configuration and vibrates in response to the sound propagating from the first sound chamber of the respective sound chambers to the second sound chamber of the respective sound chambers; and
   forming an external housing fully enclosing the medical device, wherein the external housing comprises an overlay cover, wherein the overlay cover is configured to vibrate in response to the sound propagating through the plurality of sound chambers in the stacked configuration.
20. The method of clause 19, wherein at least a first sound tuning hole is separated from the speaker by a first air volume, wherein the first air volume is adjacent to the speaker and initiates a sound propagation pathway, a second sound tuning hole is separated from the first sound tuning hole by a second air volume, a first diaphragm and a third air volume, the first diaphragm being in contact with the second air volume and the third air volume and configured to vibrate, and the second sound tuning hole is separated from a second diaphragm by a fourth air volume, the fourth air volume being in contact with the second diaphragm, and wherein the second diaphragm comprises the overlay cover.
21. The method of any of clauses 19 or 20, wherein each of the plurality of sound chambers includes respective constrained air volumes, and wherein each of the respective diaphragms is configured to vibrate within the respective constrained air volumes.
22. The method of any of clauses 19-21, wherein forming at least one of the plurality of diaphragms comprises forming at least one of the plurality of diaphragms using polyetherimide (PEI).
23. The method of any of clauses 19-22, wherein forming the overlay cover comprises using liquid silicone rubber (LSR).
24. The method of any of clauses 19-23, wherein forming at least one of the plurality of the diaphragms comprises forming at least one of the plurality of diaphragms in a circular shape.
25. The method of clause 24 or of any of clauses 19-24, wherein forming at least one of the plurality of the diaphragms in a circular shape comprises forming at least one of the plurality of diaphragms to have a diameter of 4 millimeters to 12 millimeters.
26. The method of any of clauses 19-25, wherein forming at least one of a plurality of diaphragms comprises forming at least one of the plurality of diaphragms to have a thickness between 0.0005 inches to 0.005 inches.
27. The method of any of clauses 19-26, wherein forming at least one of the plurality of sound chambers in the stacked configuration comprises forming one of the plurality of sound chambers in the stacked configuration as a plurality of sound tuning holes arranged in a circular configuration.
28. The method of any of clauses 19-27, wherein the first diaphragm fully seals the medical device to achieve a watertight medical device.
29. The method of any of clauses 19-28, wherein the medical device has an ingress protection rating of IP28, IP38, IP48, IP58, IP68, IPX8, IPX7, IPX6, or IPX5.
30. The method of any of clauses 19-29, wherein forming the external housing comprises forming a thermal bonding adhesive, wherein the thermal bonding adhesive is applied to seal the first diaphragm to the external housing, and wherein the thermal bonding adhesive geometry includes an arched bridge.

## Claims

1. A medical device comprising:
a speaker comprising a vibrating membrane and configured to generate a sound that propagates through the medical device;
a plurality of sound chambers in a stacked configuration and configured to propagate the sound generated by the speaker through one or more sound tuning holes of the sound chambers, wherein in the stacked configuration the sound propagates serially through the plurality of sound chambers;
one or more diaphragms, within the medical device, configured to vibrate in response to the sound propagating though the medical device, wherein each of the one or more diaphragms separates respective sound chambers in the stacked configuration and vibrates in response to the sound propagating from a first sound chamber of the respective sound chambers to a second sound chamber of the respective sound chambers;
an external housing fully enclosing the medical device and comprising an overlay cover, wherein the overlay cover is configured to vibrate in response to the sound propagating through the plurality of sound chambers in the stacked configuration.

2. The medical device of claim 1, wherein at least a first sound tuning hole is separated from the speaker by a first air volume, wherein the first air volume is adjacent to the speaker and initiates a sound propagation pathway, a second sound tuning hole is separated from the first sound tuning hole by a second air volume, a first diaphragm and a third air volume, the first diaphragm being in contact with the second air volume and the third air volume and configured to vibrate, and the second sound tuning hole is separated from a second diaphragm by a fourth air volume, the fourth air volume being in contact with the second diaphragm, and wherein the second diaphragm comprises the overlay cover.

3. The medical device of any of claims 1 or 2, wherein each of the plurality of sound chambers includes respective constrained air volumes, and wherein each of the respective diaphragms is configured to vibrate within the respective constrained air volumes.

4. The medical device of any of claims 1-3, further comprising:
processing circuitry configured to:
determine that an audible alert is to be output; and
upon determining that the audible alert is to be output, cause the speaker to generate the sound that propagates through the medical device through the plurality of sound chambers in the stacked configuration.

5. The medical device of any of claims 1-4, wherein the speaker is a surface-mounted piezoelectric device,
and/or
wherein a material of at least one of the one or more diaphragms is polyetherimide (PEI) or liquid silicone rubber (LSR),
and/or
wherein a material of the overlay cover is liquid silicone rubber (LSR),
and/or
wherein at least one of the one or more diaphragms is circular, particularly wherein at least one of the one or more diaphragms has a diameter between approximately 4 millimeters to 12 millimeters.

6. The medical device of any of claims 1-5, wherein at least one of a one or more diaphragms has a thickness between 0.0005 inches to 0.005 inches,
and/or
wherein the medical device is a handheld insulin pump device.

7. The medical device of any of claims 1-6, wherein at least one of the plurality of sound chambers in the stacked configuration comprises a plurality of the sound tuning holes in a circular configuration.

8. The medical device of any of claims 1-7, wherein the first diaphragm fully seals the medical device to achieve a watertight medical device,
and/or
wherein the medical device has an ingress protection rating of IP28, IP38, IP48, IP58, IP68, IPX8, IPX7, IPX6, or IPX5.

9. The medical device of any of claims 1-8, wherein a thermal bonding adhesive is applied to seal the first diaphragm to the external housing, and wherein the thermal bonding adhesive geometry includes an arched bridge,
and/or
wherein the sound tuning holes are axially misaligned.

10. A method of generating an audible alert comprising:
determining that an audible alert is to be output; and
upon determining that the audible alert is to be output, causing a speaker comprising a vibrating membrane to generate a sound that propagates through the medical device through a plurality of sound chambers in a stacked configuration and configured to propagate the sound generated by the speaker through one or more sound tuning holes of the sound chambers, wherein in the stacked configuration the sound propagates serially through the plurality of sound chambers, wherein the sound chambers in the stacked configuration are separated by one of one or more diaphragms and each of the one or more diaphragms vibrates in response to the sound propagating from a first sound chamber of the respective sound chambers to a second sound chamber of the respective sound chambers, wherein an external housing fully encloses the medical device and comprises an overlay cover, wherein the overlay cover is configured to vibrate in response to the sound propagating through the plurality of sound chambers in the stacked configuration,
particularly
wherein the medical device comprises the features of any of claims 1-9.

11. A method of manufacturing a medical device, the method comprising:
forming a first sound chamber of a plurality of sound chambers in a stacked configuration, wherein the plurality of sound chambers is configured to propagate a sound generated by a speaker through one or more sound tuning holes of sound chambers, wherein in the stacked configuration the sound propagates serially through the plurality of sound chambers;
forming a first of a plurality of diaphragms within the medical device;
forming a second sound chamber of the plurality of sound chambers;
wherein each of the plurality of diaphragms is configured to vibrate in response to the sound propagating though the respective sound chambers and one of the one or more diaphragms separates respective sound chambers in the stacked configuration and vibrates in response to the sound propagating from the first sound chamber of the respective sound chambers to the second sound chamber of the respective sound chambers; and
forming an external housing fully enclosing the medical device, wherein the external housing comprises an overlay cover, wherein the overlay cover is configured to vibrate in response to the sound propagating through the plurality of sound chambers in the stacked configuration.

12. The method of claim 11, wherein at least a first sound tuning hole is separated from the speaker by a first air volume, wherein the first air volume is adjacent to the speaker and initiates a sound propagation pathway, a second sound tuning hole is separated from the first sound tuning hole by a second air volume, a first diaphragm and a third air volume, the first diaphragm being in contact with the second air volume and the third air volume and configured to vibrate, and the second sound tuning hole is separated from a second diaphragm by a fourth air volume, the fourth air volume being in contact with the second diaphragm, and wherein the second diaphragm comprises the overlay cover,
and/or
wherein each of the plurality of sound chambers includes respective constrained air volumes, and
wherein each of the respective diaphragms is configured to vibrate within the respective constrained air volumes.

13. The method of any of claims 11-12, wherein forming at least one of the plurality of diaphragms comprises forming at least one of the plurality of diaphragms using polyetherimide (PEI),
and/or
wherein forming the overlay cover comprises using liquid silicone rubber (LSR),
and/or
wherein forming at least one of the plurality of the diaphragms comprises forming at least one of the plurality of diaphragms in a circular shape, particularly wherein forming at least one of the plurality of the diaphragms in a circular shape comprises forming at least one of the plurality of diaphragms to have a diameter of 4 millimeters to 12 millimeters,
and/or
wherein forming at least one of a plurality of diaphragms comprises forming at least one of the plurality of diaphragms to have a thickness between 0.0005 inches to 0.005 inches.

14. The method of any of claims 11-13, wherein forming at least one of the plurality of sound chambers in the stacked configuration comprises forming one of the plurality of sound chambers in the stacked configuration as a plurality of sound tuning holes arranged in a circular configuration,
and/or
wherein forming the external housing comprises forming a thermal bonding adhesive, wherein the thermal bonding adhesive is applied to seal the first diaphragm to the external housing, and
wherein the thermal bonding adhesive geometry includes an arched bridge.

15. The method of any of claims 11-14, wherein the first diaphragm fully seals the medical device to achieve a watertight medical device,
and/or
wherein the medical device has an ingress protection rating of IP28, IP38, IP48, IP58, IP68, IPX8, IPX7, IPX6, or IPX5.
